(19) Europäisches Patentamt
European Patent Office
Office européen des brevets

(11) Veröffentlichungsnummer: **0 351 640**
**A1**

(12) **EUROPÄISCHE PATENTANMELDUNG**

(21) Anmeldenummer: **89112244.2**

(51) Int. Cl.4: **C07C 271/28 , C08G 18/38**

(22) Anmeldetag: **05.07.89**

(30) Priorität: **16.07.88 DE 3824287**

(43) Veröffentlichungstag der Anmeldung:
**24.01.90 Patentblatt 90/04**

(84) Benannte Vertragsstaaten:
**CH DE FR GB IT LI NL**

(71) Anmelder: **BAYER AG**

**D-5090 Leverkusen 1 Bayerwerk(DE)**

(72) Erfinder: **Sanders, Josef, Dr. c/o Mobay Corporation**
**Mobay Road**
**Pittsburgh P.A. 15205(US)**
Erfinder: **Dieterich, Dieter, Dr.-Prof.**
**Henry-Theodor-von-Böttinger-Strasse 16**
**D-5090 Leverkusen 1(DE)**

(54) **N,N-disubstituierte Oligo-und Polyurethane, Verfahren zu ihrer Herstellung sowie ihre Verwendung bei der Herstellung von Kunststoffen.**

(57) Die vorliegende Erfindung betrifft neue N,N-disubstituierte Oligo- und Polyurethane mit Hydroxylendgruppen, ein Verfahren zu ihrer Herstellung sowie ihre Verwendung bei der Herstellung von Kunststoffen.

EP 0 351 640 A1

## N,N-disubstituierte Oligo- und Polyurethane, Verfahren zu ihrer Herstellung sowie ihre Verwendung bei der Herstellung von Kunststoffen

Die Erfindung betrifft neue N,N-disubstituierte Oligo-und Polyurethane mit Hydroxylendgruppen, ein Verfahren zu ihrer Herstellung sowie ihre Verwendung bei der Herstellung von Kunststoffen.

Es ist bekannt, daß N,N-disubstituierte Urethane im Vergleich zu N-monosubstituierten Urethane eine größere Thermofestigkeit und Hydrolysestabilität aufweisen. Da N,N-disubstituierte Urethane nicht durch Polyaddition von Diolen an Diisocyanate zugänglich sind, erfolgt ihre Herstellung im allgemeinen durch Polykondensation von Bischlorkohlensäureestern mit disekundären Aminen in Gegenwart von Basen (vgl. hierzu Houben-Weyl, Bd. E20, Teil 2, Thiema-Verlag Stuttgart S. 1580, 1710). Nachteilig dabei sind die begrenzte Verfügbarkeit und der vergleichsweise hohe Preis der disekundären Amine sowie die schlechte Reproduzierbarkeit der gewünschten mittle ren Molgewichte. Außerdem sind nach diesem Verfahren nur Produkte mit Aminoendgruppen zugänglich, die für viele Anwendungszwecke Isocyanaten gegenüber eine unerwünscht hohe Reaktivität aufweisen. N,N-disubstituierte Urethane sind daher bislang ohne technisches Interesse geblieben.

Aufgabe der vorliegenden Erfindung war es, ein wirtschaftliches Verfahren zur Herstellung N,N-disubstituierter Urethane zu entwickeln, das die beschriebenen Nachteile vermeidet.

Es wurde nun gefunden, daß man neue, Hydroxylendgruppen aufweisende N,N-disubstituierte Polyurethane herstellen kann, indem man NCO-Gruppen-terminierte Verbindungen mit gegebenenfalls in 3-Stellung substituierten Oxetanmethanolen umsetzt, die resultierenden Urethan-und Oxetangruppen aufweisenden Verbindungen durch Umsetzung mit Alkylierungsmitteln und Metallhydroxiden vollständig oder teilweise an den Urethanstickstoffen alkyliert und schließlich durch Addition von starken Säuren an die Oxetangruppen unter Ringöffnung Hydroxylendgruppen erzeugt.

Gegenstand der vorliegenden Erfindung sind somit neue N,N-disubstituierte Urethangruppen und Hydroxylendgruppen aufweisende Verbindungen der allgemeinen Formel

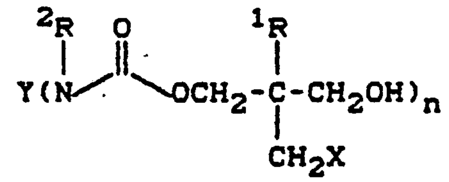

in welcher

X den nach Dissoziation des Protons bzw. Protonen verbleibenden Rest einer Starken ein- bzw. mehrbasigen Säure oder eine Hydroxylgruppe, bevorzugt ein Chlor-, Brom- oder Jodatom bedeutet,

Y einen n-wertigen, bevorzugt zweiwertigen, gegebenenfalls durch Sauerstoff-, Schwefel-, Siliziumatome, Ester-, Carbonat-, Harnstoff-, N-monosubstituierte und/oder N,N-disubstituierte Urethangruppen unterbrochenen Kohlenwasserstoffrest des Molgewichts 15 - 8000, bevorzugt 300 - 4000 bedeutet,

$^1R$ einen Kohlenwasserstoffrest des Molgewichts 15 -200, bevorzugt einen Methyl- oder Ethylrest bedeutet,

$^2R$ den Rest eines Alkylierungsmittels, bevorzugt einen Methyl-, Ethyl- oder Benzylrest bedeutet und

n eine ganze Zahl von 1 bis 6, vorzugsweise 2 bedeutet.

Gegenstand der Erfindung ist auch ein Verfahren zur Herstellung von N,N-disubstituierten Urethangruppen und Hydroxylendgruppen aufweisenden Verbindungen durch Umsetzung von

a) (i) n-wertige, Isocyanatendgruppen aufweisenden Verbindungen der allgemeinen Formel

$Z(NCO)_n$

mit (ii) gegebenenfalls in 3-Stellung substituierten Oxetanmethanolen der allgemeinen Formel

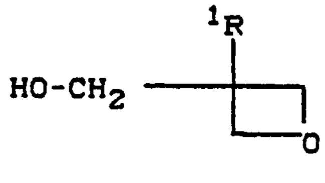

zu Verbindungen der allgemeinen Formel

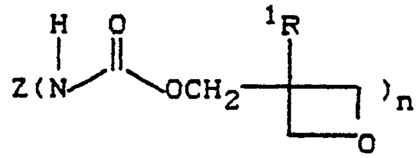

b) anschließende Umsetzung der gemäß a) erhaltenen Oxetanendgruppen aufweisenden Verbindungen mit (iii) Alkylierungsmitteln in Gegenwart von Metallhydroxiden zu vollständig oder teilweise an den Urethanstickstoffen substituierten Verbindungen der allgemeinen Formel

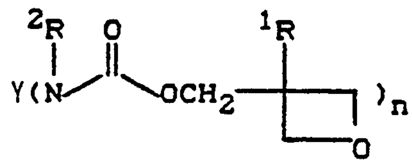

und schließlich

c) Umsetzung der zuletzt genannten Verbindungen mit starken Säuren zu Hydroxylendgruppen aufweisenden Verbindungen der allgemeinen Formel

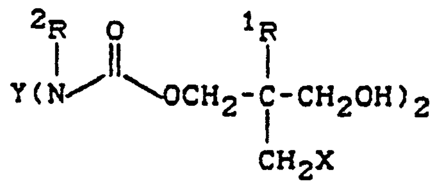

wobei in diesen Formeln x, y, $^1R$, $^2R$ und n die oben genannte Bedeutung haben und Z einen n-wertigen, bevorzugt zweiwertigen, gegebenenfalls durch Sauerstoff-, Schwefel-, Siliziumatome, Ester-, Carbonat-, Harnstoff-und/oder N-monosubstituierte Urethangruppen unterbrochenen Kohlenwasserstoffrest des Molgewichts 15 -8000, bevorzugt 300 - 4000 bedeutet.

Die Erfindung betrifft ferner die Verwendung der letztgenannten N,N-disubstiuierte und Hydroxylendgruppen aufweisenden Verbindungen bei der Herstellung von Kunststoffen, insbesondere von Polyurethankunststoffen nach dem Isocyanat-Polyadditionsverfahren.

Ausgangsmaterial (i) zur Herstellung der erfindungsgemäßen Verbindungen sind Isocyanate der allgemeinen Formel

$Z(NCO)_n$

in der Z und n die oben genannte Bedeutung haben. In Frage kommen beispielsweise aliphatische, araliphatische und aromatische Mono- und Polyisocyanate wie sie z.B. in Ullmann Encyklopädie der technischen Chemie, 4. Auflage, Bd. 19, S. 303 - 304 beschrieben sind. Z.B. Isocyanato-ethan, -propan, -butan, -pentan, -hexan, 6-Chlorhexylisocyanat, Isocyanatocyclohexan, Benzylisocyanat, Tetramethylendiisocyanat; Hexamethylendiisocyanat, Dekamethylendiisocyanat; 1,3-Di-(3-isocyanatopropoxy)-2,2-dimethylpropan; Cyclohexandiisocyanat-(1,4); Methylcyclohexandiisocyanat-(2,4); Methylcyclohexandiisocyanat-(2,6); 1,3-Diisocyanatocyclohexan, Gemische aus Methylcyclohexandiisocyanat-(2,4) und Methylcyclohexandiisocyanat-(2,6); Dicyclohexylmethan-4,4'-diisocyanat; 1-Isocyanato-3-isocyanatomethyl-3,5,5-trimethylcyclohexan (Isophorondiisocyanat); 1,2-Di-(Isocyanatomethyl)-cyclobutan; m- und p-Xylylendiisocyanat sowie $\alpha,\alpha,\alpha',\alpha'$-Tetramethyl-m-und/oder -p-xylylendiisocyanat oder -hexahydroxylylendiisocyanat, Phenylisocyanat, Toluolisocyanat, 1,3- und ,4-Phenylendiisocyanat, 2,5-und 2,6-Toluylendiisocyanat sowie beliebige Gemische dieser Isomeren, Diphenylmethan-2,4'-und/oder -4,4'-diisocyanat, 1,5-und 2,4-Naphthylendiisocyanat, Alkylbenzoldiisocyanate gemäß EP 0 58 368, Triphenylmethan-4,4' ,4''-triisocyanat, Polyphenyl-polymethylen-polyisocyanate, wie sieh durch Anilin-Formaldehyd-Kondensation und anschließende Phosgenierung erhalten und z.B. in dem britischen Patentschriften 874 430 und 848 671 beschrieben werden, m- und p-Isocyanatophenylsulfonyl-isocyanate gemäß der amerikanischen Patentschrift 3 454 606, perchlorierte Arylpolyisocyanate, wie sie z.B. in der deutschen Auslegeschrift 1 157 601 (amerikanische Patentschrift 3 277 138) beschrieben werden, Carbodiiumidgruppen aufweisende Polyisocyanate, wie sie in der deutschen Patentschrift 1 092 007 (amerikanische Patentschrift 3 152 162) beschrieben werden, Diisocyanate, wie sie in der amerikanischen Patentschrift 3 492 330 beschrieben werden, Allophanatgruppen aufweisende Polyisocyanate, wie sie z.B. in der britischen Patentschrift 994 890, der belgischen Patentschrift 761 626 und der veröffentlichten holländischen Patentanmeldung 7 102 524 beschrieben werden, Isocyanuratgruppen aufweisende Polyisocyanate, wie sie z.B. in der amerikanischen Patentschrift 3 001 973, in den deutschen Patentschriften 1 022 789, 1 222 067 und 1 027 394 sowie in den deutschen Offenlegungschriften 1 929 034 und 2 00 048 beschrieben werden, Urethangruppen aufweisende Polyisocyanate, wie sie z.B. in der belgischen Patentschrift 752 261 oder in der amerikanischen Patentschrift 3 394 164 beschrieben werden, acylierte Harnstoffgruppen aufweisende Polyisocyanate gemäß der deutschen Patentschrift 1 230 778, Biuretgruppen aufweisende Polyisocyanate, wie sie z.B. in der Deutschen Patentschrift 1 101 394 (amerikanische Patentschriften 3 124 605 und 3 201 372) sowie in der britischen Patentschrift 889 050 beschrieben werden, durch Telomerisationsreaktionen hergestellte Polyisocyanate, wie sie z.B. in der amerikanischen Patentschrift 3 654 106 beschrieben werden, Esterguppen aufweisende Polyisocynate, wie sie zum Beispiel in den britischen Patentschriften 965 474 und 1 072 956, in der amerikanischen Patentschrift 3 567 763 und in der deutschen patentschrift 1 231 688 genannt werden, Umsetzungsprodukte der obengenannten Isocyanate mit Acetalen gemäß der deutschen Patentschrift 1 072 385, polymere Fettsäurereste enthaltende Polyisocynanate gemäß der amerikanischen Patentschrift 3 455 883.

Es ist auch möglich, die bei der technischen Isocyanatherstellung anfallenden Isocyanatgruppen aufweisenden Destillationsrückstände, gegebenenfalls gelöst in in einem oder mehreren der vorgenannten Polyisocyanate, einzusetzen. Ferner ist es möglich, beliebige Mischungen der vorgenannten Polyisocyanate zu verwenden.

Ebenfalls geeignete Isocyanate (i) sind sogenannte NCO-Prepolymere, wie sie zum Beispiel nach bekannten Verfahren durch Polyaddition von Polyaminen, Polyhydroxylverbindungen oder, weniger bevorzugt, Polythiolen mit überschüssigen Mengen an Polyisocyanaten hergestellt werden können. Dabei kann das durchschnittliche Molgewicht der NCO-Prepolymeren über das Verhältnis der eingesetzten NCO-Gruppen zu eingesetzten NH-, OH- oder SH-Gruppen in weiten Grenzen variiert werden (Vgl. D. Dieterich, Angewandte Macromolekulare Chemie 1979, 76/77, 1114, S. 79 - 107).

Zur Herstellung der NCO-Prepolymeren sind mehrwertige, bevorzugt zweiwertige aliphatischen, araliphatische und aromatischen Isocyanate geeignet, wie sie bereits beispielhaft oben aufgeführt wurden.

Geeignete Polyamine, die mit den Diisocyanaten zur Reaktion gebracht werden können, sind beispielsweise Äthylendiamin, 1,4-Tetramethylendiamin, 1,11-Undecanmethylendiamin, 1,12-Dodecamethylendiamin sowie deren Gemische, 1-Amino-3,3,5-trimethyl-5-amino-methylcyclohexan ("Isophorondiamin"), 2,4- und 2,6-Hexahydrotoluylendiamin sowie deren Gemische, Perhydro-2,4′- und 4,4′-diaminodiphenylmethan, p-Xyllylendiamin, Bis-(3-aminopropoyl)-methylamin, Diamino-perhydroanthrazene (DE-Offenlegungsschrift 2 638 731) und cycloaliphatische Triamine gemäß DE-Offenlegungsschrift 2 614 244. Auch Hydrazin und substituierte Hydrazine, z.B. Methylhydrazin, N,N′-Dimethylhydrazin und deren Homologe sowie Säuredihydrazide kommen erfindungsgemäß in Betracht, z.B. Carbodihydrazid, Oxalsäuredihydrazid, die Dihydrazide von Malonsäure, Bernsteinsäure, Glutarsäure, Adipinsäure, β-Methyladipinsäure, Sebazinsäure, Hydracrylsäure und Terephthalsäure; Semicarbazido-alkylenhydrazide wie z.B. β-Semicarbazidopropionsäurehydrazid (DE-Offenlegungschrift 1 770 591), Semicarbazidoalkylencarbazinester wie z.B. 2-Semicarbazidoäthylcarbazinester (DE-Offenlegungschrift 1 918 504) oder auch Amino-semicarbazid-Vrbindungen wie z.B. β-Aminoethyl-semicarbazido-carbonat (DE-Offenlegungschrift 1 902 931). Zur Steuerung ihrer Reaktivität können die Aminogruppen ganz oder teilweise durch Aldimin bzw. Ketimin-Gruppen blockiert sein (US-Patentschrift 3 734 894; DE-Offenlegungschrift 2 637 115).

Als Beispiele für aromatische Diamine seien Bisanthranilsäureester gemäß den DE-Offenlegungsschriften 2 040 644 und 2 160 590, 3,5- und 2,4-Diaminobenzoesäureester gemäß DE-Offenlegunsschrift 2 025 900, die in den DE-Offenlegunsschriften 1 803 635 (US-Patentschriften) 3 681 290 und 3 736 350, 2 040 650 und 2 160 589 beschriebenen estergruppenhaltigen Diamine, die Ethergruppen aufweisenden Diamine gemäß DE-Offenlegunsschriften 1 770 525 and 1 809 172 (US-Patentschriften 3 654 364 und 3 736 295), gegebenenfalls in 5-Stellung substituierte 2-Halogen-1,3-Phenylendiamine (DE-Offenlegunsschriften 2 001 772, 2 025 896 und 2 065 869), 3,3′-Dichlor-4,4′-diamino-diphenylmethan, Toluylendiamin, 4,4′-Diaminodiphenylmethan, 4,4′,-Diaminodiphenyldisulfide (DE-Offenlegungsschrift 2 404 976), Diamonodiphenyldithioäther (DE-Offenlegunsschrift 2 509 404, durch Alkylthiogruppen substituierte aromatische Diamine (DE-Offenlegungsschrift 2 638 760), Diaminobenzolphosphorsäureester (DE-Offenlegungschrift 2 459 491), Sulfonat- oder Carboxylatgruppen enthaltende aromatische Diamine (DE-Offenlegunsschrift 2 720 166) sowie die in der DE-Offenlegungsschrift 2 635 400 aufgeführten hochschmelzenden Diamine genannt. Beispiele für aliphatischaromatische Diamine sind die aminoalkylthioaniline gemäß DE-Offenlegungsschrift 2

734 574.

Geeignete Polyhydroxylverbindungen zur Herstellung der NCO-Prepolymeren sind vorzugsweise Diole mit einem mittleren Molekulargewicht von 60 - 3000, insbesondere 60 - 1000. In Frage kommen beispielweise niedermolekulare Polyole wie Äthylenglykol, Propylenglykol-(1,2) und -(1,3), Butylenglykol-(1,4) und --(2,3), Pentandiol(1,5), Hexandiol-(1,6), Octanidol-(1,8), Neopentyglykol, 1,4-Bis-hydroxymethyl-cyclohexan, 2-Methyl-1,3-propandiol, Dibrombutendiol (US-Patentschrift 3 723 392), Glyzerin, Trimethylpropan, Hexantriol-(1,2,6), Trimethyloläthan, Pentaerythrit, Chinit, Mannit und Sorbit Ricinusöl, Diathylenglykol, Triäthylenglykol, Tetraäthylenglykol, höhere Polyäthylenglykole mit einem Molekulargewicht bis 400, Dipropylenglykol, höhere Polypropylenglykole mit einem Molekulargewicht bis 400, Dibutylenglykol, höhere Polybutylenglykole mit einem Molekulargewicht bis 400, 4,4′-Dihydroxy-diphenylpropan, Dihydroxymethylhydrochinon, Äthanolamin, Diäthanolamin, N-Methyldiätanolamin, Triäthanolamin und 3-Aminopropanol.

Als niedermolekulare Polyole kommen erfindungsgemäß auch die Gemische von Hydroxyaldehyden und Hydroxyketonen ("Formose") bzw. die hieraus durch Reduktion erhaltenen mehrwertigen Alkohole ("Formit") in Frage, wie sie bei der Selbstkondensation von Formaldehydhydrat in Gegenwart von Metallverbindungen als Katalysator und von zur Endiolbildung befähigten Verbindungen als Co-Katalysator entstehen (DE-Offenlegunsschriften 2 639 084, 2 714 104, 2 721 186, 2 738 154 und 2 738 512). Um Kunststoffe mit verbesserter Flammwidrigkeit zu erhalten, setzt man diese Formosen mit Vorteil in Kombination mit Aminoplastbildern und/oder Phosphiten ein (DE-Offenlengungsschriften 2 738 513 und 2 738 532). Auch Lösungen von Polyisocyanatpolyadditionsprodukten, insbesondere von ionische Gruppen aufweisenden Polyurethanharnstoffen und/oder von Polyhydrazodicarbonamiden, in niedermolekularen, mehrwertigen Alkoholen kommen erfindungsgemäß als Polyolkomponente in Betracht (DE-Offenlegunsschrift 2 638 759.

Geeignete Polyhydroxylverbindungen zu Herstellung von NCO-Prepolymeren sind ferner höhermolekulare Verbindungen mit einem mittleren Molgewicht von 400 -3000, vorzugsweise 400 - 1000, welche mindesten 2 bis 6, vorzugsweise 2 reaktive Hydroxylgruppen pro Molekül aufweisen.

In Frage kommen beispielsweise die in der Polyurethanchemie üblichen, hydroxylgruppentragenden Polyacetale, Polythioether, Polycarbonate, Polyamide, Polysiloxane und/oder Polybutadiene, Polyester, Polylactone sowie Polyester; insbesondere aber Hydroxylgruppen tragende Polyether.

Die erfindungsgemäß in Frage kommenden Hydroxylgruppen aufweisenden Polyether sind solche der an sich bekannten Art und werden z.B. durch Polymerisation von Epoxiden wie Ethylenoxid, Propylenoxid, Butylenoxid, Tetrahydrofuran, Styroloxid oder Epichlorhydrin mit sich selbst, z.B. in Gegenwart von $BF_3$, oder durch Anlagerung dieser Epoxide, gegebenenfalls im Gemisch oder nacheinander, an Startkomponenten mit reaktionsfähigen Wasserstoffatomen wie Wasser, Alkohole oder Amine, z.B. Ethylenglykol, Propylenglykol-(1,3) oder -(1,2), Trimethylolpropan, 4,4′-Dihydroxy-diphenylpropan, Anilin, Ammoniak, Ethanolamin oder Ethylendiamin hergestellt. Auch Sucrosepolyether, wie z.B. in den deutschen Auslegeschriften 1 176 358 und 1 064 938 beschrieben werden, kommen erfindungsgemäß in Frage. Vielfach sind solche Polyether bevorzugt, die überwiegend (bis zu 90 Gew.-%, bezogen auf alle vorhandenen OH-Gruppen im Polyether) primäre OH-Gruppen aufweisen. Auch durch Vinylpolymerisate modifizierte Polyether, wie sie z.B. durch Polymerisation von Styrol und Acrylnitril in Gegenwart von Polyethern entstehen (amerikanische Patentschriften 3 383 351, 3 304 273, 3 523 093, 3 110 695, deutsche Patentschrift 1 152 536), sind geeignet, ebenso OH-Gruppen aufweisende Polybutadiene.

Als Polyacetale kommen z.B. die aus Glykolen, wie Dioder Tri-ethylenglykol, 4,4′-Dihydroxyethoxydiphenylmethan, Hexandiol und Formaldehyd oder durch Polymerisation cyclischer Acetale, wie z.B. Trioxan, herstellbare Verbindungen in Frage. Als Hydroxylgruppen aufweisende Polycarbonate kommen solche der an sich bekannten Art in Betracht, die z.B. durch Umsetzung von Diolen wie Propandiol-1,6, Di-, Tri- oder Tetraethylenglykol oder Thiodiglykol mit Diarylcarbonaten, z.B. Diphenylcarbonat, oder Phosgen hergestellt werden können (DE-B 1 694 080, 1 915 908 und 2 221751; DE-A 2 605 024).

Als Polyester aus Dicarbonsäuren und Diolen kommen solche aus Adipinsäure- und Isophthalsäure und geradkettigen und/oder verzweigten Diolen in Betracht, ebenso Lactonpolyester, vorzugsweise auf Basis von Caprolacton und Starterdiolen.

Unter den Polythioethern seien insbesondere die Kondensationsprodukte von Thiodiglykol mit sich selbst und/oder mit anderen Glykolen angeführt.

Auch bereits Urethan- oder Harnstoffgruppen enthaltende Polyhydroxylverbindungen sowie gegebenenfalls modifizierte natürliche Polyole sind verwendbar. Auch Anlagerungsprodukte von Alkylenoxiden an PhenolFormaldehyd-Harze oder auch an Harnstoff-Formaldehyd-Harze sind erfindungsgemäß einsetzbar. Es ist auch möglich, z.B. gemäß DE-A 2 559 372, in die Polyhydroxylverbindungen Amidgruppen einzuführen.

Erfindungsgemäß können gegebenenfalls auch Polyhydroxylverbindungen eingesetzt werden, in welchen hochmolekulare Polyaddukte bzw. Polykondensate oder Polymerisate in feindisperser oder gelöster Form enthalten sind. Derartige Polyhydroxylverbindungen werden z.B. erhalten, wenn man Polyadditionsreaktionen (z.B. Umsetzungen zwischen Polyisocyanaten und aminofunktionellen Verbindungen) bzw. Polykondensationsreaktionen (z.B. zwischen Formaldehyd und Phenolen und/oder Aminen) in situ in den o.g., Hydroxylgruppen aufweisenden Verbindungen ablaufen läßt. Derartige Verfahren sind beispielsweise in den DE-B 1 168 075 und 1 260 142, sowie den DE-A 2 324 134, 2 423 984, 2 512 385, 2 513 815, 2 550 796, 2 550 797, 2 550833, 2 550 862, 2 633 293 und 2 639 254 beschrieben. Es ist aber auch möglich, gemäß US-PS 3 869 413 bzw. 2 550 860 eine fertige wäßrige Polymerdispersion mit einer Polyhydroxylverbindung zu vermischen und anschließend aus dem Gemisch das Wasser zu entfernen.

Auch durch Vinylpolymerisate modifizierte Polyhydroxylverbindungen, wie sie z.B. durch Polymerisation von Styrol und Acrylnitril in Gegenwart von Polyethern (US-PS 3 383 351, 3 304 273, 3 523 093, 3 110 695, DE-B 1 152 536) oder Polycarbonatpolyolen (DE-PS 1 769 795, US-PS 3 637 909) enhalten werden, sind für das erfindungsgemäße Verfahren geeignet. Bei Verwendung von Polyetherpolyolen, welche gemäß DE-A 2 442 101, 2 644 922 und 2 646 141 durch Pfropfpolymerisation mit Vinylphosphonsäureestern sowie gegebenenfalls (Meth)acrylnitril, (Meth)acrylamid oder OH-funktionellen (Meth)acrylsäureestern modifiziert wurden, erhält man Kunststoffe von besonderer Flammwidrigkeit.

Geeignete, wenn auch weniger bevorzugte Polyhydroxylkomponenten (i) sind auch organofunktionelle Polysiloxane, die zwei endständige, gegenüber Isocyanatgruppen reaktionsfähige Gruppen und Struktureinheiten der Formel $-O-Si(R)_2-$ aufweisen, wobei in dieser Formel R für einen $C_1$-$C_4$-Alkyl- oder einen Phenylrest, vorzugsweise jedoch für einen Methylrest, steht. Erfindungsgemäß eigen sich als Ausgangsmaterialien sowohl die an sich bekannten, endständige organofunktionelle Gruppen aufweisenden reinen Polysiloxane als auch die an sich bekannten endständig organofunktionellen Siloxanpolyoxyalkylencopolymeren.

In Frage kommen auch weniger bevorzugte Organopolysiloxane der allgemeinen Formel

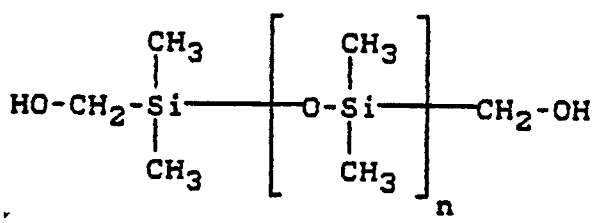

n = 5 bis 29

Sie werden in an sich bekannter Weise durch Äquilibrierung von 1,1,2,3-Tetramethyl-1,3-hydroxymethyldisiloxan der Formal

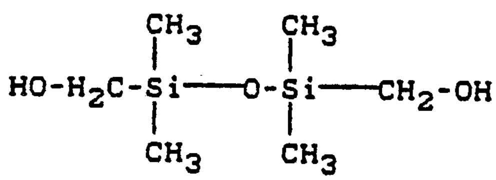

mit Octamethylcyclotetrasiloxan in Gegenwart von Schwefelsäure bzw. nach dem Verfahren der DE-B 1 236 505 hergestellt.

Selbstverständlich können zur Herstellung der Ausgangsverbindungen (i) auch Gemische der aufgeführten Isocyanate, Polyamine, Polythiole und Polyhydroxylverbindungen eingesetzt werden. Ihre Umsetzung kann nach dem üblichen Isocyanat-Polyadditionsverfahren gegebenenfalls in Gegenwart von in der Polyurethanchemie üblichen Katalysatoren und Hilfsstoffen in Substanz oder bevorzugt in Gegenwart von organischen Lösungsmitteln erfolgen, in welchen auch die nachfolgende Urethanalkylierung (Stufe b) des Verfahrens) durchgeführt wird,

Besonders bevorzugte Isocyanate (i) sind Isocyanatendgruppen aufweisende Prepolymere des Molgewichts 400 -4,000 auf Basis handelsüblicher Diisocyanate des Molgewichts 100 - 400 und Alkandiolen, Polyetherdiolen oder Polycarbonatdiolen.

Zu den erfindungsgemäß in Betracht kommenden Ausgangsmaterialien (ii) gehören in 3-Stellung substituierte Oxetanmethanole der allgemeinen Formel

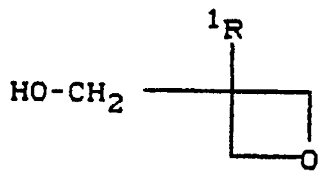

in der
$^1R$ einen Kohlenwasserstoffrest des Molgewichts 15 - 200 bedeutet.

In Frage kommen beispielsweise 3-Tolyl-, 3-Phenyl-, 3-Benzyl-, 3-Hexyl-. 3-Pentyl-, 3-Butyl-, 3-Propyl- und vorzugsweise 3-Ethyl- und 3-Methyl-oxetanmethanol.

Bei der Durchführung der Stufe a) des erfindungsgemäßen Verfahrens wird die Menge an Ausgangsmaterial (II) in der Regel so bemessen, daß für jede NCO-Gruppe der Ausgangsverbindung (i) ein Molekül (ii) zur Verfügung steht. Die bei dieser Umsetzung resultierenden Produkte weisen dementsprechend in der Regel keine freien NCO-Gruppen mehr auf.

Die Umsetzung der erfindungsgemäßen Ausgangsmaterialien (i) und (ii) kann in Substanz oder zweckmäßig in einem organischen Lösungsmittel, gegebenenfalls in Gegenwart von in der Polyurethanchemie üblichen Katalysatoren und Hilfsstoffen erfolgen. In der Regel werden Lösungsmittel bevorzugt, in welchen auch die nachfolgende Urethanalkylierung (Stufe b) des Verfahrens) durchgeführt werden kann.

Geeignete organische Lösungsmittel sind beispielsweise: Benzol, Toluol, Xylol, Chlorbenzol, Dichlorbenzol, Trichlorbenzol, Diethylether, Diisopropylether, tert.-Butylmethylether, Tetrahydrofuran, Dioxan, Ethylenglykoldimethylether, Essigester, Aceton, Methylethylketon, Acetonitril, Furfurol, Methylenchlorid, Chloroform, Trichlorethylen, Tetrachlorethylen, Nitromethan, Nitropropan. Bevorzugt werden polare aprotische Lösungsmittel wie zum Beispiel Dimethylformamid, Dimethylacetamid, N-Methylpyrrolidon, Tetramethylharnstoff, N-Methylcaprolactam, Dimethylsulfoxid, Tetramethylensulfon, Hexamethylenphosphorsäuretriamid usw. Ganz besonders bevorzugt werden Dimethylformamid, Dimethylsulfoxid und N-Methylpyrrolidon.

Selbstverständlich können auch beliebige Gemische derartiger Lösungsmittel eingesetzt werden.

Die Menge des Lösungsmittels wird hierbei im allgemeinen so bemessen, daß sie ausreicht, um die Ausgangsmaterialian (i) und (ii) klar zu lösen. In der Praxis bedeutet dies, daß die Lösungsmittel im allgemeinen in einer Menge von 50 bis 1,000 bevorzugt 100 bis 500 Gew.-Teilen Lösungsmittel pro 100 Gew.-Teilen des Gemisches aus den Komponenten (i) und (ii) zum Einsatz gelangen.

Die Umsetzung wird im allgemeinen bei einer Temperatur von 0 - 140 °C, bevorzugt bei 20 - 90 °C mit Überdruck, Unterdruck oder zweckmäßig drucklos, kontinuierlich oder diskontinuierlich durchgeführt.

Zur Durchführung der Stufe a) des erfindungsgemäßen Verfahrens kann man beispielsweise so vorgehen, daß man zunächst die Ausgangskomponente (i) im gewählten Lösungsmittel vorlegt (bzw. zweckmäßig bereits in diesem Lösungsmittel herstellt), die Ausgangskomponente (ii), gegebenenfalls im betreffenden Lösungsmittel gelöst, zugibt und anschließend gegebenenfalls bei erhöhter Temperatur solange nachrührt, bis IR-spektrokopisch in der Reaktionsmischung kein Isocyanat mehr nachgewiesen wird.

Die resultierende Produktlösung wird zweckmäßig direkt in die Stufe b) des erfindungsgemäßen Verfahrens eingesetzt.

In Stufe b) des Verfahrens werden die Zwischenprodukte gemäß Stufe a) des Verfahrens mit (iii) Alkylierungsmitteln umgesetzt.

Zum Einsatz kommen beispielsweise Verbindungen der Formel

$^2RA$

$R^2$ einen aromatischen Kohlenwasserstoffrest mit 6 -18, bevorzugt 6 - 13 C-Atomen,
einen aliphatischen Kohlenwasserstoffrest mit 1 -18, vorzugsweise1 - 12 C-Atomen
einen cycloaliphatischen Kohlenwasserstoffrest mit 7 - 30, vorzugsweise 7 - 15 C-Atomen oder
einen araliphatischen Kohlenwasserstoffrest mit 7 -39, vorzugsweise 7 - 15 C-Atomen bedeutet
und in der

A eine geeignete Abgangsgruppe wie z.B. ein Halogenidion, eine Sulfat-, Sulfonat-, Phosphatoder Phosphonatgruppe ist.

Selbstverständlich darf der Kohlenwasserstoff $R^2$ neben der Abgangsgruppe A auch weitere funktionelle Gruppen tragen, wenn diese unter den erfindungsgemäßen Reaktionsbedingungen inert sind oder in definierter Weise mit den erfindungsgemäßen Reagenzien reagieren, z.B. Nitro-, bestimmte Ester-, Urethan-Amidgruppen und Sulfonylgruppen, nicht aktiviertes, aromatisch gebundenes Halogen, Epoxidgruppen, Aziridingruppen, Ethergruppen, Thioethergruppen und dergleichen mehr.

Als Alkylierungsmittel kommen beispielsweise in Frage:

Methylchlorid, -bromid, Ethyl-chlorid, -bromid, Propylchlorid, -bromit, i-Propyl-chlorid, -bromid, n-Butychlorid, -bromid, iso-Butyl-chlorid, -bromid, Cyclohexyl-chlorid, -bromid, Octyl-, Noyl-, Decyl-, Undecyl-, Dodecyl-chlorid und bromid, Benzyl-chlorid, -bromid, Allyl-chlorid, -bromid, p-Nitrobenzyl-chlorid, -bromid, 2,4-Dinitrochlorbenzol, 2,4-Dinitrofluorbenzol, 2,4,6-Trinitrochlorbenzol, 2,4,6-Trinitrofluorbenzol, Dimethylsufat, Diethylsulfat, p-Toluolsufonsäuremethylester, -ethylester, Ethylenchlorhydrin, Ethylenbromhydrin, Epichlorhydrin.

Selstverständlich können auch Mischungen dieser Alkylierungsmittel eingesetzt werden.

Besonders bevorzugte Alkylierungsmittel sind Benzylchlorid, -bromid sowie Methylchlorid und -bromid.

Erfindungsgemäß wird die Stufe b) des Verfahrens in Gegenwart von Metallhydroxiden durchgeführt. In Frage kommen beispielsweise Alkalimetallhydroxide wie Kaliumoder Natriumhydroxid. Aus wirtschaftlichen Gründen wird Natriumhydroxid bevorzugt. Selbstverständlich kann man z.B. auch Lithium-, Rubidium- und Barimhydroxid oder auch feuchtes Silberoxid einsetzen. Es kann in machen Fällen vorteilhaft sein, Mischungen dieser Metallhydroxide einzusetzen.

Es kann in machen Fällen vorteilhaft sein, die Umsetzung in Gegenwart eines Phasentransferkatalysators durchzuführen. Solche Katalysatoren werden z.B. in E.V. und S. S. Dehmlow, Phase Transfer Catalysis, 2. Auflage, Verlag Chemie 1983, beschrieben. Geeignete Katalysatoren sind quarternäre Ammonium- oder Phosphoniumsalze der Formel

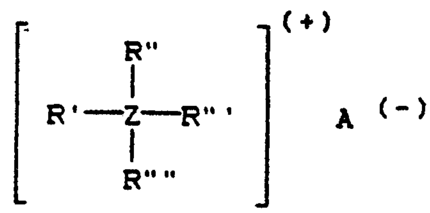

in welcher

Z für Stickstoff oder Phosphor steht,

R', R'', R''' und R'''' für gleiche oder verschiedene Reste stehen und Alkylgruppen mit 1 bis 18 Kohlenstoffatomen bedeuten, wobei einer der Reste auch für einen araliphatischen Rest mit 7 bis 15 Kohlenstoffatomen stehen kann, und wobei die Summe der Kohlenstoffatome der view Reste vorzugsweise bei 12 bis 31 liegt.

Typische Beispiele geeigneter Katalysatoren sind N-Benzyl-N,N,N-trimethyl- und N- Benzyl-N,N,N-triethyl-ammoniumchlorid oder -bromid, N- Benzyl-N-dodecyl-N,N-dimethyl-ammoniumchlorid oder -bromid, N,N,N,N-Tetra-n-hexyl-ammoniumchlorid oder -bromid, N-Benzyl-N,N,N-tri-n-octyl-ammoniumchlorid oder -bromid oder die diesen Ammoniumsalzen entsprechenden Phosphoniumsalze.

Die beispielhaft genannten quarternären Ammonium- oder Phosphoniumslaze werden bei der Durchführung des erfindungsgemäßen Verfahrens vorzugsweise in Substanz oder in Form ihrer wäßrigen Lösungen (beispielsweise mit einen Feststoffgehalt von 30 bis 60 Gew.-%) und vorzugsweise in einer Menge von 1 bis 10 Mol-%, bezogen auf die Molzahl der vorhandenen Urethangruppen eingesetzt.

Bei Verwendung der erfindungsgemäß bevorzugte polaren aprotischen Lösungsmittel wie z.B. Dimethylformamid, N-Methylpyrrolidon oder Dimethylsulfoxid kann ohne Nachteile auf den Zusatz von Phasentransferkatalysatoren verzichtet werden.

Bei der Durchführung der Stufe b) des erfindungsgemäßen Verfahrens können die Alkylierungsmittel (iii) bezogen auf die in den Zwischenprodukten gemäß Stufe a) des Verfahrens enthaltenen Urethangruppen sowohl in stöchiometrischer Menge als auch im Über- oder Unterschuß zum Einsatz gelangen. Dabei sind gegebenenfalls die bereits in der Ausgangskomponente (i) enthaltenen Urethangruppen mit zu berücksichtigen. Selbstverständlich wird bei Verwendung eines Unterschusses an Alkylierungsmittel nur eine Teilalkylierung erreicht werden, während die Verwendung eines größeren Überschusses an Alkylierungsmittel unwirtschaftlich ist.

Vorzugsweise wird die Komponente (iii) so bemessen, daß auf jedes Mol in den Zwischenprodukten gemäß Stufe a) enthaltenen Urethangruppen 1 bis 1,5 Mol der Komponente (iii) vorliegt. Der bei der Reaktion frei werdende Halogenwasserstoff kann, wie schon erwähnt, durch Zusatz von Metallhydroxiden gebunden werden. Dabei wird ihre Menge so bemessen, daß sie zumindest zur Neutralisation des abgespaltenen Halogenwasserstoffs ausreicht. Besonders bevorzugt werden sie in einer Menge verwendet, daß 1 bis 3 Mol Basenäquivalente pro Mol Hydroxylgruppen zur Verfügung stehen.

Wie schon erwähnt wird die Stufe b) des erfindungsgemäßen Verfahrens bevorzugt in einem organischen Lösungsmittel durchgeführt. In Betracht kommen bei-spielsweise die bei der Beschreibung der Stufe

a) des Verfahrens aufgeführten Lösungsmittel in den dort beschriebenen Mengen.

Die Stufe b) erfindungsgemäßen Verfahrens wird im allgemeinen bei 10 bis 100 °C, bevorzugt bei 20 bis 60 °C mit Überdruck, Unterdruck oder zweckmäßig drucklos, kontinuierlich oder diskontinuierlich durchgeführt.

Bei Einsatz leicht flüchtiger Alkylierungsmittel wie z.B. Methylchlorid, -bromid oder Ethylchlorid ist es zweckmäßig, die Umsetzung in einem Autoklaven unter Druck durchzuführen.

Dabei kann entweder in einem aprotischen organischen Lösungsmittel oder in überschüssigem, verflüssigtem Alkylierungsmittel, gegebenenfalls in Gegenwart eines Phasentransferkatalysators, gearbeitet werden.

Die Verweilzeit beträgt im allgemeinen 0,5 bis 24 Stunden; bevorzugt werden 0,5 bis 8 Stunden.

Zur Durchführung der Stufe b) des erfindungsgemäßen Verfahrens kann man beispielsweise so vorgehen, daß man die Ausgangsmaterialien und gegebenenfalls den Phasentransferkatalysator im gewählten Lösungsmittel vorlegt und die Base in gelöster oder suspendierter Form, bevorzugt in fester, möglichst fein gemahlener Form, unter Rühren, gegebenenfalls unter Kühlung portionsweise oder kontinuierlich zugibt. Anschließend rührt man bei Raumtemperatur oder gegebenenfalls erhöhter Temperatur so lange nach, bis IR-spektroskopisch ein vollständiger Umsatz der anfangs vorhandenen Urethangruppen angezeigt wird und/oder dünnschicht- oder gaschromatographisch kein Ausgangsstoff (iii) mehr nachgewiesen werden kann.

Die Aufarbeitung der Reaktionsmischung erfolgt in an sich bekannter Weise. Zweckmäßig verdünnt man das Reaktionsgemisch mit einem mit Wasser nicht mischbaren inerten Lösungsmittel, wäscht mit Wasser oder Salzlösung neutral, destilliert die Lösungsmittel gegebenenfalls im Vakuum ab und trocknet im Vakuum. Die Neutralisation des Reaktionsgemisches kann auch durch Behandeln mit $CO_2$ erfolgen. Als inerte Lösungsmittel kommen beispielsweise Toluol, Methylenchlorid, Chlorbenzol, Dichlorbenzol, 1,2-Dichlorethan, Trichlorethylen u.a. in Betracht. Das so erhaltene Rohprodukt kann im allgemeinen ohne weitere Reinigung weiterverarbeitet werden. Sollte es jedoch infolge unvollständigen Umsatzes noch geringe Mengen an Ausgangstoff (ii) enthalten, kann dieser zweckmäßig durch Dünnschichtdestillation entfernt werden.

Grundsätzlich denkbar, jedoch weniger bevorzugt, wäre auch eine Arbeitsweise, die darin besteht, das in der Stufe b) anfallende Reaktionsgemisch, gegebenenfalls nach Neutralisation überschüssigen Alkalihydroxids ohne Zwischenisolierung direkt der Stufe c) zuzuführen. Die in der Stufe b) des erfindungsgemäßen Verfahrens erhaltenen vollständig oder teilweise alkylierten, Oxetanendgruppen aufweisenden Verbindungen werden in der Stufe c) des Verfahrens mit starken Säuren zu Hydroxylendgruppen aufweisenden Verbindungen umgesetzt. In Frage kommen sowohl starke ein- oder mehrbasige organische als auch anorganische Säuren. Beispielsweise können eingesetzt werden: Trifluor- und Trichloressigsäure, Methan-, Chlormethan-, Ethan-, 2-Chlorethan-, Propan-, Butan-, Perfluorbutan-, 4-Chlorbutan-, Perfluoethan-, 2-Hydroxyethan-, Benzol-, 2-, 3- und 4-Chlorbenzol-, 2,5-und 3,4-Dichlor-, 2-, 3- und 4-Nitrobenzol-, 4-Chlor-3-nitrobenzol-, 2-Chlor-5-nitrobenzol-, 2,4-Dinitrobenzol-, 4-Methylbenzol-, 1-Naphthalin-, 5-Nitro-1-naphthalin-, 1,3-Benzoldi-, 5-Nitro-1,3-benzoldi-, 1,5-, 1,6-, 2,6-und 2,7-Naphthalindi-, /1,1′-Bisphenyl/-4,4′-di-, sowie 2-, 3-und 4-Hydroxylbenzolsulfonsäure, anorganische Säuren wie zum Beispiel Salpeter-, Phosphor-, Schwefel-, Perchlor-, Chlor-, Perbrom-, Brom-, Perjod-, Jod- und Flußsäure sowie bevorzugt Chlorwasserstoff-, Bromwasserstoff- und Jodwasserstoffsäure.

Bei der Durchführung der Stufe c) des erfindungsgemäßen Verfahrens kann die Säure bezogen auf die in den Zwischenprodukten gemäß Stufe b) des Verfahrens enthaltenen Oxetangruppen sowohl in stöchiometrischer Menge als auch im Über- oder Unterschuß zum Einsatz gelangen. Selbstverständlich wird bei Verwendung eines Säureunterschusses nur ein Teil der vorhandenen Oxetangruppen unter Ringöffnung und Ausbildung von Hydroxylgruppen umgesetzt werden, während die Verwendung eines größeren Überschusses an Säure unwirtschaftlich ist. Vorzugsweise wird die Säuremenge so bemessen, daß auf jedes Mol in den Zwischenprodukten gemäß Stufe b) des Verfahrens enthaltenen Oxetangruppen 1 bis 2 Mol Säure vorliegt.

Die Stufe c) des erfindungsgemäßen Verfahrens wird bevorzugt in einem organischen Lösungsmittel durchgeführt. In Betracht kommen beispielsweise die bereits bei der Beschreibung der Stufen a) und b) des Verfahrens aufgeführten Lösungsmittel in den dort beschriebenen Mengen. Bevorzugte Lösungsmittel sind Diisopropylether, Ethylenglykoldimethyl- und -diethylether, Tetrahydrofuran und Dioxan. Die Stufe c) des erfindungsgemäßen Verfahrens wird im allgemeinen bei 10 - 100 °C, bevorzugt bei 20 bis 60 °C mit Überdruck, Unterdruck oder zweck mäßig drucklos, kontinuierlich oder diskontinuierlich durchgeführt. Die Verweilzeit beträgt im allgemeinen 0,5 - 24 Stunden, bevorzugt werden 0,5 bis 8 Stunden.

Zur Durchführung der Stufe c) des erfindungsgemäßen Verfahrens kann man beispielsweise so vorgehen, daß man die Zwischenprodukte gemäß Stufe b) des Verfahrens im gewählten Lösungsmittel

vorlegt und die Säure in fester oder gasförmiger, bevorzugt in gelöster Form, gegebenenfalls unter Kühlung portionsweise oder kontinuierlich zugibt. Anschließend rührt man bei Raumtemperatur oder gegebenenfalls erhöhter Temperatur solange nach, bis zum Beispiel der titrimetrisch bestimmte Säureverbrauch vollständigen Umsatz anzeigt.

Die Aufarbeitung der Reaktionsmischung erfolgt in an sich bekannter Weise. Zweckmäßig verfährt man wie bei der Beschreibung der Stufe b) des Verfahrens angegeben.

Die nach dem erfindungsgemäßen Verfahren herstellbaren Hydroxylendgruppen aufweisenden N,N-disubstituierten Oligo- bzw. Polyurethane sind wertvolle Ausgangsstoffe für die Herstellung thermostabiler Kunststoffe. Insbesondere zeigen die erfindungsgemäßen Hydroxylgruppen aufweisenden N,N-disubstituierten Urethane größere thermische, thermooxidative und photooxidative Stabilität (vgl. R. Vieweg, A. Höchtlen, Kunststoff Handbuch Bd. VII, Polyurethane, Hanser Verlag München 1966, S. 11, S. 21) sowie ein günstigeres Brandverhalten als die entsprechenden N-monosubstituierten Urethane. Sie eignen sich als Reaktionspartner für gegebenenfalls blockierte Polyisocyanate bei der Herstellung von Polyurethanen (Polyurethanharnstoffen), gegebenenfalls zelligen Polyurethankunststoffen oder Polyurethanschaumstoffen, wobei sie gegebenenfalls auch mit anderen niedermolekularen (Molekulargewicht 32 bis 399) und/oder höhermolekularen (Molekulargewicht 400 bis ca. 12.000 Verbindungen mit gegenüber Isocyanaten reaktiven Gruppen kombiniert werden können. Geeignete Ausgangskomponenten für die Herstellung von Polyurethankunststoffen werden beispielsweise in DE-A 2 302 564, DE-A 2 432 764 (US-PS 3 903 679) sowie in den DE-A 2 639 083, 2 512 385, 2 513 815, 2 550 796, 2 550 797, 2 550 833, 2 550 860 und 2 550 862 genannt. Dort finden sich auch Hinweise auf bei der Polyurethanherstellung gegebenenfalls mitzuverwendenden Hilfs- und Zusatzstoffe.

Die Herstellung von Polyurethankunststoffen mittels der erfindungsgemäßen Polyhydroxylverbindungen ist ebenfalls Gegenstand der vorliegenden Erfindung. Ihre Anwendung kann z.B. für Elastomere, Beschichtungen, Fäden in der Applikation aus Schmelzen, Lösungen, Dispersionen oder als Reaktivkomponentenmischung erfolgen. In Betracht kommen natürlich auch alle anderen an sich bekannten Anwendungsbereiche für höhermolekulare Polyhydroxylverbindungen.

Die nachfolgenden Beispiele dienen zur weiteren Erläuterung der Erfindung. Alle Prozentangaben beziehen sich auf Gewichtsprozente. Die mittlere Korngröße des eingesetzten gepulverten Alkalihydroxides liegt bei 6 bis 9 µm.

## Beispiele

### Beispiel 1

a) Herstellung des Ausgangsurethans mit Oxetanendgruppen

Zu einer Lösung von 59 g (0,5 Mol) 1,6-Hexandiol in 645,4 g frisch destilliertem DMF tropft man unter Rühren bei Raumtemperatur 194,4 g (0,6 Mol) 2,4-Diisocyanatoalkylbenzolgemisch gemäß EP 0058368 über 2 Stunden zu und rührt 80 Minuten bei 80 °C nach. Anschließend gibt man 23,2 g (0,2 Mol) 3-Ethyl-3-oxetanmethanol zu und rührt solange bei 80 °C nach, bis IR-spektroskopisch kein Isocyanat mehr nachweisbar ist.
Aussehen: klare, gelbliche Lösung mit ca. 30 % Feststoffgehalt
Auslaufviskosität : 14 s ( 4mm Fordbecher, 25 °C)

b) Urethanalkylierung

In einem Autoklaven werden zu einer vorgelegten Dispersion von 26,4 g (0,66 Mol) gepulvertem Natriumhydroxid in 333 g (0,43 Mol Urethan) 30 %iger Urethanlösung gemäß Beispiel 1a) 27,8 g (0,55 Mol) Methylchlorid zügig zugedrückt. Anschließend wird erst 3 Stunden bei Raumtemperatur, dann noch 3 Stunden bei 60 °C nachgerührt. Die abgekühlte und entspannte Reaktionsmischung wird mit ca. 1 l Chlorbenzol verdünnt, filtriert und mit Wasser neutral gewaschen. Nach Trocknen über Natriumsulfat wird das Lösungsmittel im Vakuum abgezogen.
Ausbeute: 105 g (quantitativ) .
Aussehen: klares, bräunliches, viskoses Öl

IR: Fehlende Banden bei 3200 - 3500 $cm^{-1}$ zeigen vollständige Substitution der Urethanprotonen an

c) Ringöffnende Umsetzung der Oxetanendgruppen mit Säure

Zu einer Lösung von 200 g methyliertem Urethan gemäß Beispiel 1b) in 450 ml Dioxan wird unter Rühren bei Raumtemperatur 37,3 g 48 %ige Bromwasserstoffsäure innerhalb von 20 Minuten zugetropft und 4 Stunden bei 60 °C nachgerührt. Die abgekühlte Reaktionsmischung wird mit ca. 900 ml Methylenchlorid verdünnt und mit Wasser neutral gewaschen. Nach Trocknen über Natriumsulfat wird das Lösungsmittel im Vakuum abgezogen.
Ausbeute: 200 g (gelbraunes Öl)
Viskosität: 4600 mPas/85 °C
OH-Zahl: 30

Beispiel 2

a) Ausgangsurethan mit Oxetanendgruppen: entspricht Beispiel 1a)

b) Urethanalkylierung

Zu einer Lösung aus 766 g (1 Mol Urethan) 30 %iger Urethanlösung gemäß Beispiel 1a) und 151,9 g (1,2 Mol) Benzylchlorid gibt man unter Rühren bei Raumtemperatur portionsweise 52 g (1,3 Mol) gepulvertes Natriumhydroxid über 1 Stunde gleichmäßig zu und rührt erst 3 Stunden bei Raumtemperatur dann 3 Stunden bei 50 °C nach. Nach Abkühlen wird analaog Beispiel 1b) aufgearbeitet.
Ausbeute: 298 g (93 % d. Theorie)
Aussehen: klares, braunes, viskoses Öl
IR: fehlende Banden bei 3200 - 3500 $cm^{-1}$ zeigen vollständige Substitution der Urethanprotonen an

c) Oxetanöffnung

269,4 g benzyliertes Urethan gemäß Beispiel 2b) und 40,2 g 48 %ige Bromwasserstoffsäure werden analog Beispiel 1c) in 550 ml Dioxan umgesetzt.
Ausbeute: 243 g (klares, braunes Harz)
Viskosität: 7000 mPas/85 °C
OH-Zahl: 28

Beispiel 3

a) Ausgangsurethan mit Oxetanendgruppen

70,8 g (0,6 Mol) 1,6-Hexandiol, 187,5 g (0,75 Mol) 4,4′-Diisocyanatodiphenylmethan und 34,8 g (0,3 Mol) 3-Ethyl-3-oxetanmethanol werden analog Beispiel 1a) in 684 g frisch destilliertem DMF umgesetzt.
Aussehen: klare, hellbraune Lösung mit 30 % Feststoffgehalt
Auslaufviskosität: 31 s (4 mm Fordbecher, 25 °C)

b) Urethanalkylierung

1000 g (1,54 Mol Urethan) 30 %ige Urethanlösung gemäß Beispiel 3a), 80 g (2Mol) gepulvertes Natriumhydroxid und 101 g (2 Mol) Methylchlorid werden analog Beispiel 1b) umgesetzt.
Ausbaute: 296 g (92 % d. Theorie)
Aussehen: klares, bräunliches, zähes Öl

IR: Fehlende Banden bei 3200 - 3500 $cm^{-1}$ zeigen vollständige Substitution der Urethanprotonen an.

c) Oxetanöffnung

261,8 g methyliertes Urethan gemäß Beispiel 3b) und 71,1 g 48 %ige Bromwasserstoffsäure werden analog Beispiel 1c) in 550 ml Tetrahydrofuran umgesetzt.
Ausbeute: 234 g (klares, braunes Harz)
Viskosität: 10.400 mPas/85° C
OH-Zahl: 40

Beispiel 4

a) Ausgangsurethan mit Oxetanendgruppen entspricht Beispiel 3a)

b) Urethanalkylierung

651 g (1Mol Urethan) 30 %ige Urethanlösung gemäß Beispiel 3a), 151,9 g (1,2 Mol) Benzylchlorid und 52 g (1,3 Mol gepulvertes Natriumhydroxid werden analog Beispiel 2b) umgesetzt.
Ausbeute: 271 g (95 % d. Theorie)
Aussehen: klares, hellbraunes, viskoses Öl
IR: Sehr schwache Banden bei 3200 - 3500 $cm^{-1}$ zeigen fast vollständige Substitution der Urethanprotonen an.

c) Oxetanöffnung

243 g benzyliertes Urethan gemäß Beispiel 4b) und 48,8 g 48 %ige Bromwasserstoffsäure werden analog Beispiel 1c) in 500 ml Dioxan umgesetzt.
Ausbeute: 191 g (klares, braunes Harz)
Viskosität: 83.630 mPas/85° C
OH-Zahl: 34

Beispiel 5

a) Ausgangsurethan mit Oxetanendgruppen

236 g (2Mol) 1,6-Hexandiol, 435 g (2,5 Mol) Toluylendiisocyanat (2,4-Isomerenanteil: 80 %) und 116 g (1 Mol) 3-Ethyl-3-oxetanmethanol werden analog Beispiel 1a) in 1836 g frisch destilliertem DMF umgesetzt.
Aussehen: klare, gelbliche Lösung mit 30 % Feststoffgehalt
Auslaufviskosität: 17 s ( 4 mm Fordbecher, 25° C)

b) Urethanalkylierung

1050 g (2 Mol Urethan) 30 %ige Urethanlösung gemäß Beispiel 5a), 108 g (2,7 Mol) gepulvertes Natriumhydroxid und 121,2 g (2,4 Mol) Methylchlorid werden analog Beispiel 1b) umgesetzt.
Ausbeute: 296 g (86 % d. Theorie)
Aussehen: klares, bräunliches, zähes Öl
IR: Fehlende Banden bei 3200 - 3500 $cm^{-1}$ zeigen vollständige Substitution der Urethanprotonen an.

c) Oxetanöffnung

250 g methyliertes Urethan gemäß Beispiel 5b) und 77 g 48 %ige Bromwasserstoffsäure werden analog Beispiel 1c) in 500 ml Dioxan umgesetzt.
Ausbeute: 202 g (klares, braunes Harz)
Viskosität: 8800 mPas/85 °C
OH-Zahl: 59

Beispiel 6

a) Ausgangsurethan mit Oxetanendgruppen

141,6 g (1,2 Mol) 1,6-Hexandiol, 243,6 g (1,4 Mol) Toluylendiisocyanat (2,4-Isomerenanteil: 80%) und 46,4 g (0,4 Mol) 3-Ethyl-3-oxetanmethanol werden analog Beispiel 1a) in 1294,8 g frisch destilliertem DMF umgesetzt.
Aussehen: klare, gelbliche Lösung mit 25 % Feststoffgehalt
Auslaufviskosität: 15 s (4 mm Fordbecher, 25 °C)

Urethanalkylierung

616 g ( 1 Mol Urethan) 25 %ige Urethanlösung gemäß Beispiel 6a), 151,9 g (1,2 Mol) Benzylchlorid und 52 g (1,3 Mol) gepulvertes Natriumhydroxid werden analog Beispiel 2b) umgesetzt.
Ausbeute: 244 g (quantitativ)
Aussehen: klares, rotbraunes, zähes Öl
IR: Sehr schwache Banden bei 3200 - 3500 $cm^{-1}$ zeigen fast vollständige Substitution der Urethanprotonen an.

c) Oxetanöffnung

205 g benzyliertes Urethan gemäß Beispiel 6b) und 34,4 g 48 %ige Bromwasserstoffsäure werden analog Beispiel 1c) in 450 ml Dioxan umgesetzt.
Ausbeute: 180 g (klares braunes Harz)
Viskosität 67.780 mPas/85 °C
OH-Zahl: 30

Beispiel 7

a) Ausgangsurethan mit Oxetanendgruppen

141,6 g (1,2 Mol) 3-Methyl-1,5-pentandiol, 235,2 g (1,4 Mol) Hexamethylendiisocyanat und 46,4 g (0,4 Mol) 3-Ethyl-3-oxetanmethanol werden analog Beispiel 1a) in 987,4 g frisch destilliertem DMF umgesetzt.
Aussehen: klare gelbliche Lösung mit 30 % Feststoffgehalt, die sich bei längerem Stehen salbenartig verfestigt
Auslaufviskosität: 17 s (4 mm Fordbecher, 25 °C)

b) Urethanalkylierung

350 g (0,7 Mol Urethan) aufgeschmolzene 30 %ige Urethanlösung gemäß Beispiel 7a), 80 g (0,84 Mol) Methylbromid und 32 g (0,8 Mol) gepulvertes Natriumhydroxid werden analog Beispiel 2b) umgesetzt.
Ausbeute: 53 g (gelbliches, viskoses Öl)
IR: Eine schwache Bande bei 3300 $cm^{-1}$ zeigt unvollständigen Umsatz an.

c) Oxetanöffnung

110 g methyliertes Urethan gemäß Beispiel 7b) und 28,7 g 48 %ige Bromwasserstoffsäure werden analog Beispiel 1c) in 300 ml Dioxan umgesetzt.

Ausbeute: 97 g (klares, gelbliches Harz)
Viskosität: 17.820 mPas/85 °C
OH-Zahl: 43

Beispiel 8

a) Ausgangsurethan mit Oxetanendgruppen

Zu einer Lösung von 300 g (2 Mol) Triglykol in 2450 g frisch destilliertem DMF tropft man unter Rühren bei Raumtemeratur 522 g (3 Mol) Diisocyanatotoluolgemisch (80 % 2,4-, 20 % 2,6-) über 3 h zu und rührt 30 Minuten bei Raumtemperatur nach. Anschließend gibt man 232 g 3-Ethyl-3-oxetanmethanol zu und rührt solange bei 35 °C nach, bis IR-spektroskopisch kein Isocyanat mehr nachweisbar ist.
Aussehen: klare, gelb-braune Lösung mit ca. 30 % Feststoffgehalt
Auslaufviskosität: 14 s (4 mm Fordbecher, 25 °C)

Urethanalkylierung

In einen Autoklaven werden zu einer vorgelegten Dispersion von 131 g (3,3 Mol) gepulvertem Natriumhydroxid in 1600 g (2,7 Mol Urethan) 30 %iger Urethanlösung gemäß Beispiel (a) 165 g (3,3 Mol) Methylchlorid zügig zugedrückt. Anschließend wird erst 3 h bei Raumtemperatur, dann noch 2 h bei 50 °C nachgerührt. Die abgekühlte und entspannte Reaktionsmischung wird mit ca. 700 ml Methylenchlorid verdünnt, filtriert und mit Wasser neutral gewaschen. Nach Trocknen über Natriumsulfat wird das Lösungsmittel im Vakuum abgezogen.
Ausbeute: 445 g (86 % d. Th.)
Aussehen: klares, bräunliches Produkt
Viskosität: 73.722 mPas/50 °C
IR: schwächere Banden bei 3200-3500 $cm^{-1}$ zeigen fast vollständige Substitution der Urethanprotonen an.
c) Ringöffnende Umsetzung der Oxetanendgruppen mit Säure SFJ 873) zu einer Lösung von 350 g (0,6 Mol OH) methyliertem Urethan gemäß Beispiel 8b) in 525 ml Dioxan wird unter Rühren bei Raumtemperatur 155 g HBr 48 % (0,9 Mol) innerhalb 1 h zugetropft, 1 h bei Raumtemperatur und 3 h bei 50 °C nachgerührt. Die abgekühlte Reaktionsmischung wird mit ca. 800 ml Methylenchlorid verdünnt, filtriert und mit Wasser neutral gewaschen. Nach Trocknen über Natriumsulfat wird das Lösungmittel im Vakuum abgezogen.
Ausbeute: 370 g (95 % d. Th.)
Aussehen: klar, braun, zäh
Viskosität: 116370 mPas/50 °C
OH-Zahl: 97

Beispiel 9

a) Ausgansurethan mit Oxetanendgruppen

268 g (2 Mol) Dipropylenglykol, 522 g (3 Mol) Diisocyanatotoluolgemisch (80 % 2,4-, 20 % 2,6-) und 232 g (2 Mol) 3-Ethyl-3-oxetanmethanol werden analog Beispiel 8a) in 2385 g frisch destilliertem DMF umgesetzt.
Aussehen: klar, gelb-braun
Auslaufviskosität: 145 s (4 mm Fordbecher, 25 °C)

b) Urethananlkylierung

1600 g (2,8 Mol Urethan) 30 %ige Urethanlösung gemäß Beispiel 9a), 136 g (3,4 Mol) gepulvertes Natriumhydroxid und 171 g (3,4 Mol) Methylchlorid werden analog Beispiel 8b) umgesetzt.
Ausbeute: 476 g (92 % d. Th.)
Aussehen: hellbraun
IR: schwächere Banden bei 3200-3500 $cm^{-1}$ zeigen fast vollständige Substitution der Urethanprotonen an.

c) Oxetanöffnung

200 g (0,36 Mol OH-) methyliertes Urethan gemäß Beispiel 9b) und 53,3 g (0,54 Mol) 37 %ige Salzsäure werden analog Beispiel 8c) in 300 ml Dioxan umgesetzt.
Ausbeute: 160 g (70 % d. Th.)
Aussehen: klar, hellbraun
Viskosität: nicht meßbar, zu hart
OH-Zahl: 99

Beispiel 10

a) Ausgangsurethan mit Oxetanendgruppen

402 g (3 Mol) Dipropylenglykol, 696 g (4 Mol) Diisocyanatotoluolgemisch (80 % 2,4-, 20 % 2,6-) und 232 g (2 Mol) 3-Ethyl-3-oxetanmethanol werden analog Beispiel 8a) in 3103 g frisch destilliertem DMF umgesetzt.
Aussehen: klar, gelb-braun
Auslaufviskosität: 14 s ( 4 mm Fordbecher, 25 °C)

b) Urethanalkylierung

1400 g (2,5 Mol Urethan) 30 %ige Urethanlösung gemäß Beispiel 10a), 121 g (3,0 Mol) gepulvertes Natriumhydroxid und 153 g (3,0 Mol) Methylchlorid werden analog Beispiel 8b) umgesetzt.
Ausbeute: 373 g (82 % d. Th.)
Aussehen: dunkelbraun
Viskosität: 7221 mPas/80%)
IR: schwächere Banden bei 3200-3500 $cm^{-1}$ zeigen fast vollständige Substitution der Urethanprotonen an.

c) Oxetanöffnung

300 g (0,42 Mol $OH^-$) methyliertes Urethan gemäß Beispiel 10 b) und 104,6 g (0,62 Mol) 48 %ige Bromwasserstoffsäure werden analog Beispiel 8c) in 450 ml Dioxan umgesetzt.
Ausbeute: 282 g (84 % d. Th.)
Aussehen: klar, hellbraun
Viskosität: 2590 mPas/50 °C
OH-Zahl: 84

Beispiel 11

a) Ausgangsurethan mit Oxetanendgruppen

Zu einer Lösung von 800 g (2 Mol) Polyglykol 400 in 2626 g frisch destilliertem DMF tropft man unter Rühren bei Raumtemperatur 2/3 von 522 g (3 Mol) Diisocyanatotoluolgemisch (80 % 2,4-, 20 % 2,6-) über

2 h zu. Hierauf gibt man 232 g (2 Mol) 3-Ethyl-3-oxetanmethanol zu und rührt 10 Minuten bei Raumtemperatur nach. Anschließend wird das restliche Diisocyanatotoluolgemisch innerhalb 1 h zugetropft und bei RT nachgerührt bis IR-spektroskopisch kein Isocyanat mehr nachweisbar ist.
Aussehen: klar, gelb-braun
Auslaufviskosität: 24 s (4 mm Fordbecher, 25° C)

b) Urethanalkylierung

1600 g (1,8 Mol Urethan) 30 %ige Urethanlösung gemäß Beispiel 11a), 80 g (2,0 Mol) gepulvertes Natriumhydroxid und 112 g (2,2 Mol) Methylchlorid werden analog Beispiel 8b) umgesetzt.
Ausbeute: 445 g (88 d. Th.)
Aussehen: klar, gelb, braun
Viskosität: 4562 mPas/50° C
IR: schwächere Banden bei 3200-3500 $cm^{-1}$ zeigen fast vollständige Substitution der Urethanprotonen an.

c) Oxetanöffnung

380 g (0,46 Mol OH-) methyliertes Urethan gemäß Beispiel 11 b) und 116 g (0,69 Mol) 48 %ige Bromwasserstoffsäure werden analog Beispiel 8c) in 500 ml Dioxan umgesetzt. Ausbeute: 348 g (84 % d. Th.)
Aussehen: klar, hellgelb
Viskosität: 10649 mPas/50° C
OH-Zahl: 75

**Ansprüche**

1. Hydroxylendgruppen aufweisende Verbindungen der allgemeinen Formel

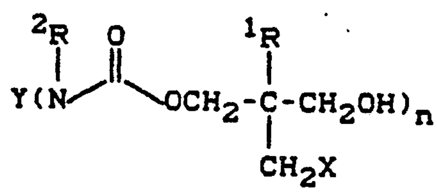

in welcher
X den nach Dissoziation des Protons bzw. der Protonen verbleibenden Rest einer starken ein-bzw. mehrbasigen Säure oder eine Hydroxylgruppe bedeutet,
Y einen n-wertigen, gegebenenfalls durch Sauerstoff-, Schwefel-, Siliziumatome, Ester-, Carbonat-, Harnstoff-, N-monosubstituierte und/oder N,N-disubstituierte Urethangruppen unterbrochenen Kohlenwasserstoffrest des Molgewichts 15 - 8000 bedeutet,
$^1R$ einen Kohlenwasserstoffrest des Molgewichts 15 - 200 oder Wasserstoff bedeutet, $^2R$ den Rest eines Alkylierungsmittels bedeutet und
n eine ganze Zahl von 1 bis 6 bedeutet.

2. Hydroxylendgruppen aufweisende Verbindungen nach Anspruch 1, dadurch gekennezeichnet, daß
X den Rest einer starken einbasigen Säure oder eine Hydroxylgruppe bedeutet,
Y einen n-wertigen, gegebenenfalls durch Sauerstoff-, Schwefel-, oder Siliziumatome, Harnstoff-, N-monosubstituierte und/oder N,N-disubstituierte Urethangruppen unterbrochenen Kohlenwasserstoffrest des Molgewichts 15 -6000 bedeutet.
$^1R$ einen Methyl- oder Ethylrest bedeutet
$^2R$ den Rest eines aliphatischen oder araliphatischen Alkylierungsmittels bedeutet und
n eine ganze Zahl von 2 bis 4 bedeutet.

3. Hydroxylendgruppen aufweisende Verbindung nach Anspruch 1 und 2, dadurch gekennzeichnet, daß
X ein Chlor-, Brom- oder Jodatom bedeutet,
Y einen zweiwertigen, gegebenenfalls durch Sauerstoffatome, N-monosubstituierte und/oder N,N-disubstituierte Urethangruppen unterbrochenen Kohlenwasserstoffrest des Molgewichts 300 - 4000 bedeutet,

$^1R$ einen Ethylrest bedeutet und

$^2R$ einen Methyl- oder Benzylrest bedeutet.

4. Verfahren zur Herstellung von Hydroxylendgruppen aufweisenden Verbindungen nach Anspruch 1 bis 3, dadurch gekennzeichnet, daß man

a) (i) n-wertige, Isocyanatendgruppen aufweisende Verbindungen der allgemeinen Formel $Z(NCO)_n$

mit (ii) gegebenenfalls in 3-Stellung substituierten Oxetanmethanolen der allgemeinen Formel

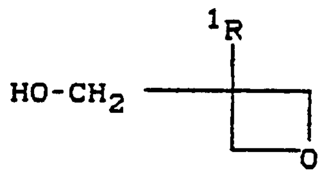

zu Verbindungen der allgemeinen Formel

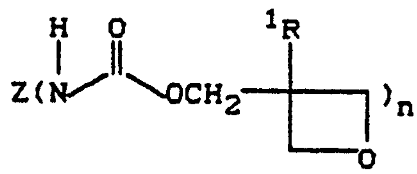

umsetzt,

b) die so erhaltenen Oxetanendgruppen aufweisenden Verbindungen durch Umsetzung mit (iii) Alkylierungsmitteln in Gegenwart von Metallhydroxiden vollständig oder teilweise an den Urethanstickstoffen zu Verbindungen der allgemeinen Formel

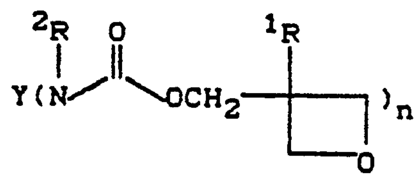

alkyliert und anschließend

c) die zuletzt genannten Verbindungen mit starken Säuren zu Hydroxylendgruppen aufweisenden Verbindungen der allgemeinen Formel

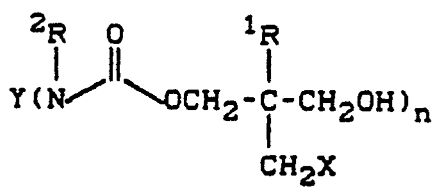

umsetzt, wobei in diesen Formeln $^1R$, $^2R$, X, Y und n die in den Ansprüchen 1 bis 3 genannte Bedeutung haben und

Z einen n-wertigen, gegebenenfalls durch Sauerstoff-, Schwefel-, Siliziumatome, Ester-, Carbonat-, Harnstoff- und/oder N-monosubstituierte Urethangruppen unterbrochenen Kohlenwasserstoffrest des Molgewichts 15 bis 8000 bedeutet.

5. Verfahren nach Anspruch 4, dadurch gekennzeichnet, daß man die Stufe b) des Verfahrens in einem organischen Lösungsmittel in Gegenwart von Natrium-und/oder Kaliumhydroxid und eines Phasentransferkatalysators durchführt.

6. Verfahren nach Anspruch 4 oder 5, dadurch gekennzeichnet, daß man die Stufe b) des Verfahrens in

Gegenwart von Natrium- und/oder Kaliumhydroxid in DMF, DMA, DMSO oder NMP als Lösungsmittel durchführt.

7. Verwendung der Hdroxylendgruppen aufweisenden Verbindungen nach Anspruch 1 bis 3 bei der Herstellung von Kunststoffen.

8. Verwendung der Hydroxylendgruppen aufweisenden Verbindungen nach Anspruch 1 bis 3 als Aufbaukomponente bei der Herstellung von Polyurethankunststoffen nach dem Isocyanat-Polyadditionsverfahren.

Europäisches Patentamt

# EUROPÄISCHER RECHERCHENBERICHT

Nummer der Anmeldung

EP 89 11 2244

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | DE-A-1 943 532 (FARBENFABRIKEN BAYER AG)<br>* Beispiele 11,14 * | 1-8 | C 07 C 271/28<br>C 08 G 18/38 |
| X | CHEMICAL ABSTRACTS, Band 68, Nr. 3, 15. Januar 1968, Seite 1965, Zusammenfassung Nr. 9630g, Columbus, Ohio, US; F. PALLOS: "Analgesics of the aromatic fluoro and pentaerythritol series", & PROMOTIONSARBEIT (ZURICH) 1963(3370), 77 pp<br>* Pentaerythritol dihutylcarbanate * | 1 | |
| A | EP-A-0 019 844 (BAYER AG)<br>* Anspruch 4 * | 1 | |

RECHERCHIERTE SACHGEBIETE (Int. Cl.5)

C 07 C 271/00
C 08 G 18/00

Der vorliegende Recherchenbericht wurde für alle Patentansprüche erstellt

| Recherchenort | Abschlußdatum der Recherche | Prüfer |
|---|---|---|
| DEN HAAG | 25-08-1989 | WELLS A.G. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)